# EUROPEAN PATENT APPLICATION

(11) **EP 2 025 746 A1**
(43) Date of publication of application: **18.02.2009**
(21) Application number: 07450139.6
(22) Date of filing: 16.08.2007
(51) Int. Cl.: C12N 5/06, C12N 5/08, A61K 39/00

(54) **Dendritic cells**

(71) Applicant: Cell Med Research GMBH, 3502 Krems-Lerchenfeld (AT)
(72) Inventor: Rubiolo, Cristina, 1220 Vienna (AT)
(74) Representative: Sonn & Partner Patentanwälte

(57) **Abstract**

The present invention relates to dendritic cells loaded with at least one nucleic acid molecule encoding a tumor associated antigen or fragment thereof and at least one proteic molecule being a tumor associated antigen or fragment thereof and methods for manufacturing the same.

## Description

The present invention relates to dendritic cells and their use.

Dendritic cells (DCs) are bone-marrow-derived antigen-presenting cells (APCs) that play a pivotal role in both induction and regulation of the immune response. It has been described that the manipulation of DCs, in order to produce a natural vaccine adjuvant can be particularly effective in stimulating anti-tumor immunity. In humans, diminished DCs function has been documented in patients with several different tumor types (breast, head and neck, colorectal and renal cell cancer, hepatocellular carcinoma and malignant melanoma). These changes have been seen mainly in blood, in cells infiltrating cancers and more recently in lymphonodes (LNs). All the studies have confirmed the defective DC function in the blood of patients as well as the substantial inhibition of DC activity in both blood and tumor-draining LNs. Moreover, it has been documented that most chemotherapies deeply impair DC function, whereas the responses of the T cells from patients are comparable with those of healthy donors, suggesting that the defects in the functional activity of DCs constitute the major factor in the inability of the host immunosystem to elicit an appropriate and beneficial anticancer immune response. The defects in the functionality of cancer patients'DCs correlate as well with an impaired production of IL-12. In addition, it was shown that the IL-10 levels in serum from patients with cancer are significantly higher than those found in normal age- and sex-matched controls. IL-10 has been shown to have a significant inhibitory effect on several aspects of DC function, such as the expression of costimulatory molecules and the ability to synthesize IL-12. Importantly, IL-10-treated DCs become tolerogenic. Moreover, in patients with progressively tumor growing, the DCs are both immature and switched off. Since the antitumor effect of DCs is dependent on their level of activation and maturation (Labeur et al., 1999), the lack of costimulatory molecules, associated with hindered maturation, is likely to result in failure of induction of a tumor-specific T cell cytotoxic response.

The most common approach to using DCs for vaccines is to prepare large numbers of autologous mature myeloid DC (MDCs) ex vivo, load them with antigens, and inject them back into the subject. The right amount of MDC can be achieved by using one of the following methods: (1) differentiating DCs from leukapheresis/elutriation-derived monocytes with GM-CSF and IL-4 or with GM-CSF and IL-13 or (2) directly isolating DCs from leukapheresis products by density gradient centrifugation or with commercially available closed systems that use immunomagnetic beads.

DCs are matured in vitro by the addition of a "cocktail" of IL-1β, IL-6, TNF-α, and PGE2 or of LPS and IFN-γ. While several groups observed that DCs matured with a cocktail including PGE2 still express CCR7 and induce Th1 as well as CD8+ T-cell responses but that they do not secrete detectable bioactive IL-12p70; DCs matured with LPS and IFN-γ secrete high levels of bioactive IL-12p70.

The increasing evidence in favor of tumor immunosurveillance supports the strategie that immunotherapies or "vaccines" may prove effective therapeutic tools against cancer. Indeed, scientific literature includes many reports that show how vaccination of cancer patients can induce immunologic and clinical responses.

It is an object of the present invention to provide dendritic cells showing enhanced immunologic properties.

These DCs should preferably show a strongly diminished capability to phagocyte (at least 5% in comparison to unloaded, smDCs), they may be able to migrate in response to stimuli, they may display high IL-12 production, without or with reduced IL-10 secretion, they may enhance T-cell cytotoxic response and they may not induce any T-reg coupled to MHC II response.

Therefore the present invention relates to a dendritic cell loaded with at least one nucleic acid molecule encoding a tumor associated antigen or fragment thereof and at least one proteic molecule being a tumor associated antigen or fragment thereof.

The DCs of the present invention show a strongly diminished capability to phagocyte (at least 5% in comparison to unloaded, smDCs), since they express already the tumor antigen on their surface; they can migrate in response to stimuli; they display high IL-12 production, without IL-10 secretion; they enhance T-cell cytotoxic response, since the antigen encoded by the nucleic acid molecule is produced as endogenous antigen by DCs and therefore complexed only through MHC I; they do not induce any T-reg coupled to MHC II response, since the antigen encoded by the nucleic acid molecule cannot be processed through MHC II and in case of phagocytosis, the tumor protein would be complexed to MHC II, but due to the contemporaneous activation of the MHC I, this event causes the activation of T-mem, without T-reg stimulation.

The double loading with a nucleic acid molecule and proteic tumor antigen ensures both a short-term response of the immune system by activating the cytotoxicity through T-cytotoxic, NK and NKT as well as a long-term response through the activation of the T-mem. In this way, the reiteration of DCs injections is not anymore required on the long period.

The DCs of the present invention are preferably loaded with universal tumor antigens (these are tumor associated antigens present in more than 2, preferably more than 5, more preferably more than 10, even more preferably more than 20, in particular more than 30, different tpyes of tumors) as well as type-specific tumor antigens and to the combination of these two types. For example, this method can be applied to a combined use of survivin and telomerase reverse transcriptase (TERT), in order to accomplish one important requirement for antigens to be used in immunotherapy: their expression must be restricted to the tumor cells, whereas it should not or only in a reduced manner be expressed or be present in healthy tissues. Indeed, although, the majority of the tumor antigens are expressed in different amount by normal cells, survivin, e.g., is exclusively expressed during embryonic and fetal development but it becomes undetectable in terminally differentiated normal adult tissue, and is re-expressed in tumor cell lines and several human cancer cells at a frequency of 34-100%.Also the TERT protein, unlikely from its RNA subunit, is expressed almost only by tumor cells, even at very early stages of tumorigenesis. As a prognostic factor, survivin and TERT expression is significantly associated with poor clinical outcome in cancers, such as breast cancer, neuro-blastoma, colorectal cancer, lung cancer and esophageal cancer. As survivin and TERT are preferentially expressed in tumor versus normal tissue, adverse effects on normal, differentiated cells are unlikely. Indeed, recent studies demonstrated that patient treated with DCs loaded with survivin RNA or TERT peptide do not develop any adverse events related to immunotherapy, other than a local reaction at the vaccination site, despite induction of impressive immune reactivity against survivin and TERT as measured by IFN-γ ELISPOT. Moreover, the strong immunological response against survivin and TERT at the time of tumor response underscores the tumor regression, following vaccination.

On the other side, the DCs of the present invention can be used to treat and/or prevent specific tumors. For example, in the case of breast cancer, DCs may be loaded with Her2-protein and Muc-1 mRNA.

Another possible combination of antigens (combination of a universal with a tumor-specific antigen) to be loaded on DCs is in the case of prostate cancer PSA-protein and survivin-mRNA.

The DCs of the present invention are thus loaded with two different types of molecules: a nucleic acid molecule and a proteinaceous molecule.

Comparative studies suggest that nucleic acid, in particular mRNA, transfection is superior to other antigen-loading techniques in generating immunopotent DCs. Moreover, loading of DCs with, e.g., mRNA is simple and effective. The ability to amplify nucleic acid molecules from microscopic amounts of tumor tissue extends the use of DC vaccination to every cancer patient.

Loading of DCs with nucleic acid-encoding defined tumor antigens is simple, reproducible, and effective. Nucleic acid molecules corresponding to gene products whose sequence is known, can be rapidly generated *in vitro* using appropriate primers and polymerase chain reaction, in the case of mRNA reverse transcriptase-polymerase chain reaction (RT-PCR), coupled to transcription reactions. Manufacture of nucleic acids for clinical use of this in vitro generated non-cell-derived product can be performed in a cost-effective and defined manner, thus streamlining and simplifying the regulatory approval process. This contrasts with the complexities and limitations of using viral vectors, which limit their availability for investigational studies and restrict their clinical evaluation. Moreover, particularly the RNA approach offers a practical solution to a common problem encountered in this kind of therapy: the fact that in most cases, it is not possible to obtain sufficient tumor tissue to generate the amount of antigens needed for an effective and sustained immunization protocol for the cancer patient. Indeed, the RNA approach allows a simple and straightforward PCR-based amplification of the antigen of interest from microscopic amounts of tumor tissue, providing a virtually inexhaustible amount of antigen. It has been shown that incubation of immature human monocyte-derived DCs with antigen mRNA in medium alone is sufficient to sensitize the DCs to stimulate an antigen-specific CTL response, comparable if not slightly superior to that associated to DCs transfected in the presence of lipid.

According to a preferred embodiment of the present invention the tumor associated antigen or fragment thereof encoded by the at least one nucleic acid molecule is selected from the group consisting of survivin and telomerase reverse transcriptase (TERT). Of course it is also possible to load dendritic cells with other universal-tumor associated antigens, such as, but not restricted to survivin and TERT, also specific tumor associated antigens, such as, but not restricted to PSA, muc-1 etc..

The dendritic cell of the present invention has several advantages over loaded dendritic cells known in the art. Since on the one hand a nucleic acid molecule encoding survivin and/or TERT and on the other hand a tumor associated antigen is used the dendritic cell of the present invention may present to the immune system an antigen which is present in many type of tumors (survivin and TERT) and at least one antigen which is present in one or more distinct type of tumors. This allows to induce an immune response which is not only highly specific to a distinct type of tumor cells but also to other tumor cells which have, for instance, not been identified at the beginning of the tumor therapy using dendritic cells loaded with a specific tumor associated antigen.

The simultaneous use of nucleic acid (e.g. RNA)/protein loading avoids the risk to trigger the T-reg response, typical of the mere protein loading and increases the beneficial effect of the nucleic acid loading by inducing a longlasting effect through the parallel activation of the cytotoxic response (through e.g. RNA) and the memory response (through protein). The use at the same time to different antigen coded by nucleic acid molecules (e.g. RNA) and protein (for example, in the breast cancer survivin-mRNA and Her-protein) may as well increase the specificity of the method and open as well new horizons in the individually patient design cell therapy.

The DCs of the present invention, loaded with e.g. survivin-mRNA simultaneously with tumor-specific antigen (protein/peptide), are not able to phagocyte , but capable to migrate in response to stimuli, such as CCL19, induce T-cell response as well as produce IL-12 over 48h in culture before and after a cycle of freezing/thawing. The product is preferably stable between -80°C and -180°C for at least 5 years and at RT or 4°C for 15'. The product can be stored in DMSO 5-10% or in Glycerol 10%.

The present invention further discloses the use of mature DCs, loaded with survivin mRNA in combination with a protein tumor antigen or fragments thereof, preferably capable to migrate but not to phagocyte, in the treatment of patients, suffering from different solid and blood cancers.

The present invention, especially its preferred embodiments, offers numerous advantages over the prior art, such as the isolation of high quantity of pure monocytes from patient's PBMC, transformation rate of peripheral monocytes (pMos) into mature DCs around 80%, GMP- and re-vivo use conform protocols, standard production in GMP conform lab, the possibility of a serum-free process, a therapy virtually applicable to any type of solid and blood tumor, availability of high quantity of phenotypic and functional mature DCs, a ready-to-use product that can be stored for years, a strongly increased cytotoxic response, a strongly increased T-mem response if the loading uses a combination of mRNA and peptide, the inhibition of the T-reg response, a technique with minimal cell manipulation, no requirement for tumor tissue/cells, the possibility of endogenous production of tumor antigen by mRNA pulsing, the combination of universal expressed and cancer-specific antigen, a high cell viability after a cycle of freezing/thawing, and the total preservation of cell functionality and phenotype after a cycle of freezing/thawing.

As used herein, the term "fragment" relates to a part or stretch of universal or type-specific tumor associated antigen, which retains at least one epitope of the wild-type protein. Consequently, a "fragment" of universal or type-specific tumor associated antigen, which maybe used to load dendritic cells binds to at least one T cell receptor, which is specific for such universal or type-specific tumor associated antigen. The "fragment" relates as well to a part or stretch of universal or type-specific tumor associated antigen, modified and synthetized *in vitro* in order to increase its immunogenic activity. The term "fragment" relates to both RNA and proteic antigen. The definition of "proteic tumor antigen" includes as well tumor exudate and lysate in addition to entire and part of proteins and peptides, isolated or chemically/recombinantly produced. According to the present invention also tumor associated antigens and fragments thereof which include amino acid modifications (deletions, substitutions, insertions) are included in the definition of tumor associated antigens and fragments thereof as used herein. This under the provision that these modified molecules still exhibit similar or even identical functionalities as the wild-type tumor associated antigen or fragment thereof.

According to a preferred embodiment of the present invention the nucleic acid molecule is a ribonucleic acid, preferably mRNA.

DCs loaded at the same time with mRNA and tumor-specific proteins/peptides completely lose the capability to phagocyte but retain the capability to migrate in response to stimuli; display high IL-12 production without IL-10 secretion; enhance T-cell cytotoxic response, since the antigen encoded by mRNA is produced as endogenous antigen by DCs and therefore complexed only through MHC I; induce T-mem response through MHC II, due to the fact that exogenous proteins can only be complexed through MHC II; do not activate the T-reg through MHC II.

The DCs are loaded with mRNA-encoding antigen for the following reasons:
- mRNA-transfected DCs are equally or more potent than peptide-pulsed DCs in stimulating CTL responses in vitro (Boczkowski, D. et al. (2000). Cancer Res 60(4): 1028-34);
- mRNA-transfected DCs are most effective APCs, capable of stimulating CD8+ responses in vitro (Gilboa, E. and J. Vieweg (2004). Immunol Rev 199: 251-63);
- mRNA transfection is so effective that it can induce and contribute to the DC maturation process ( Weissman, D. et al. (2000). J Immunol 165(8): 4710-7 and Heiser, A. et al. (2002). J Clin Invest 109(3): 409-17);
- mRNA can be loaded on the DCs via passive transfection with high efficiency, as measured as CTL response (Nair, S. K. (1998). Gene Ther 5(11): 1445-6).

Although the mRNA and nucleic acid molecule loading method shows several advantages on other loading techniques, this approach has as well an Achilles' heel: although it triggers a potent CTL response through the activation of CD8⁺ cells, it cannot induce a CD4⁺ response, even if such a response has been shown critically for the tumor immunity. Indeed, CD4⁺ cells provide important functions for the expansion and persistence of CD8⁺ CTLs, stimulate the innate arm of the immune system at the tumor site, and inhibit local angiogenesis. An optimal anti-tumor immune response therefore requires the concomitant activation of both the CD8⁺ and the CD4⁺ T-cell arms of the immune response.

In order to achieve this goal, a second loading step, along with mRNA loading approach is introduced: proteic antigen loading by passive incubation. DCs stimulated by mRNA incubation, show an increased capability, for example, to phagocyte short peptides. Therefore, it turned out that the use of the double-loading approach in order to induce both CD8⁺ and CD4⁺ T-cell arms of the immune response is advantageous. This approach constitutes a great improvement of the DCs-based cancer immune therapy, since it can, at the same time, induce the short-time response by activation of the CD8⁺ and the long-lasting memory response, through the activation of the CD4⁺ T-cell.

The double loading approach has several advantages on other methods, known in the art:
- specific activation of both the cytotoxic and the memory arms of the immune system;
- absence of the T-reg response;
- use of different antigens to direct the activation of each of the immune system arm with consequent gain in both sensitivity and strenght of the stimulus;
- therapy virtually applicable to any type of solid and blood tumor;
- combination of universal and type-specific tumor antigens;
- total conformity to GMP- and re-vivo use;
- ready-to-use product that can be stored for years;
- minimal cell manipulation;
- no requirement for tumor tissue/cells;
- combination of the positive effects coming from endogenous production of tumor antigen (encoded by the mRNA) and from endocyted proteic antigen
- high cell viability after a cycle of freezing/thawing
- total preservation of cell functionality and phenotype after a cycle of freezing/thawing
- versatility of the method.

According to another preferred embodiment of the present invention the nucleic acid molecule further comprises full-length, wild-type mRNA, a fragment of the wild-type mRNA, full-length, *in vitro* modified mRNA, a fragment of the *in vitro* modified mRNA, a mix of two or more full-length, fragments, wild-type or modified mRNAs.

According to a preferred embodiment of the present invention, the proteic antigen comprises full-length, wild-type antigen; a fragment of the wild-type antigen; full-length, *in vitro* modified antigen; a fragment of the *in vitro* modified antigen; a mix of two or more full-length, fragments, wild-type or modified antigen; full-length or fragments of the antigen coupled with xenoantigens, such as but not only Keyhole Limpet Hemocyanin (KLH); tumor lysate and peptides, isolated or chemically/recombinantly produced. The term proteic antigen comprises as well mix of antigen protein, peptide and lysate.

The dendritic cell production of the present invention may be loaded with any kind of tumor associated antigen. However, the tumor associated antigen is preferably a specific tumor associated antigen selected from the group consisting of carcinoembryonic antigen (CEA), mucin (MUC), prostate-specific membrane antigen (PSMA), BRCA and ras, and/or a universal tumor associated antigen selected from the group consisting of survivin and TERT.

According to a preferred embodiment of the present invention the dendritic cell is of human origin.

The dendritic cells of the present invention are preferably based on the expansion of autologous DCs from a human individual's peripheral blood. PBMCs are collected through leukoapheres- is or elutriation. The apheresate is processed in order to obtain monocytes (Mos). This way to obtain Mos from individuals ensures both high purity and large amounts of DCs precursors that can be therefore cultured immediately, without the need of intermediate steps, such enrichment through the use of beads, which are on the one hand, not GMP-conform and on the other hand, may be a source of contamination. The Mos are thereafter differentiated into DCs in a GMP-conform laboratory, with a culture medium, not only GMP-, but as well re-vivo conform and serum-free.

The DCs are preferably loaded when they are still immature, preferentially at first with mRNA and after with the proteic antigen. It is as well possible to perform the double loading at the same time or at first with the proteic antigen and thereafter with the mRNA.

After the loading the cells can be matured by using any of the currently available maturing cocktails, but preferentially by the use of Ribomunyl and INF-γ.

Only loaded, mature DCs are injected in the patients.

Another aspect of the present invention relates to a method for manufacturing a dendritic cell loaded with at least one nucleic acid molecule encoding a tumor associated antigen or fragment thereof and at least one proteic molecule being a tumor associated antigen or fragment thereof, preferably according to the present invention, comprising the steps of:
- providing a dendritic cell and
- loading said cell with at least one nucleic acid molecule encoding a tumor associated antigen and at least one proteic molecule being a tumor associated antigen or fragment thereof as described above.

According to a preferred embodiment of the present invention the dendritic cell is provided by transforming monocytes into dendritic cells, preferably into immature dendritic cells.

The dendritic cells may be isolated directed from a sample or by transforming isolated monocytes into immature dendritic cells. Protocols for direct isolation of dendritic cells and transformation of monocytes into dendritic cells are known in the art.

The present protocol ensures that more than 80% of the initial Mos become DCs, whereas current protocols guarantee about 10-40%. The DCs are finally matured *in vitro* in order to increase the levels of the costimulatory molecules and to trigger a strong and durable tumor-specific T cell cytotoxic response. The maturation-cocktail according to the present invention is completely GMP-conform and ensures a prolonged IL-12 production, without IL-10 secretion in comparison with the currently employed methods. Moreover, these DCs are able to produce high levels of IL-12 and no or very less amounts of IL-10 after repeated freezing/thawing cycles and remain active over at least five years if frozen in 5-10% solution of DMSO or Glycerol. At the same time, these cells keep steady their capability to migrate in response to stimuli, such as but not only CCL19, induce T-cell proliferation, respond to cytokines, show a mature phenotype and retain a viability rate over 85%. The final product undergoes a series of quality controls that ensure both its purity and its immune activity. The vaccines are preferentially stored in glycerol, a freezing medium that makes the product ready-to-use by preserving its quality for a long period, that is GMP-conform and that has no side effects for the patients. This way of storage grants as well the long-lasting activity (IL-12 secretion, T-cells stimulation and migration) of the product. Double loaded DCs loose their capability to phagocyte but retain their capability to migrate in response to stimuli and to a very lower degree, to migrate spontaneously, display high IL-12 production without IL-10 secretion; and enhance T-cell cytotoxic and T-mem response, without triggering the activation of the T-reg.

According to another preferred embodiment of the present invention the loaded immature dendritic cells are matured.

The loaded dendritic cells are preferably matured by adding ribomunyl and INF-γ.

Another aspect of the present invention relates to dendritic cells obtainable by the method according to the present invention.

Yet another aspect of the present invention relates to a pharmaceutical preparation comprising dendritic cells according to the present invention.

The dendritic cells of the present invention may be provided in a pharmaceutical preparation. Said preparation may be employed in the treatment or prevention of cancer in an individual, virtually suffering from any type of solid and blood cancer. The double loaded, mature DCs are weekly inoculated preferentially but not exclusively intradermally, in a dose of at least 10 millions DCs. The therapy is made up of a number of injections comprised between 2 and 20. The product is preferentially but not exclusively stored in 10% glycerol, thawed for a max of 15' at RT and immediately administered.

According to a preferred embodiment of the present invention the administration of the product can be accompanied by the administration of immunostimmulatory agents and/or adjuvants. Its usage is as well suitable in concomitance with chemotherapy as well as during the pauses between chemotherapic cycles.

A further aspect of the present invention relates to the use of dendritic cells according to the present invention for the manufacture of a vaccine for treating or preventing cancer in an individual.

The dendritic cells of the present invention which are loaded with at least one nucleic acid molecule encoding for a tumor associated antigen (see above) and with at least one proteic molecule being a tumor associated antigen (see above) may be suitable used to treat an individual suffering from cancer or to prevent that an individual develops cancer.

The present invention is further illustrated by the following figures and example, however, without being restricted thereto.

Fig. 1 shows the expression of survivin and PSA in immature, semi-mature, unloaded DCs and mature, survivin mRNA- as well as survivin mRNA + peptide loaded DCs, cultured as described in the examples.

Fig. 2 shows the phenotype of immature, semi-mature, unloaded DCs and mature, survivin mRNA- as well as survivin m-RNA + peptide loaded DCs, cultured as described in the examples.

Fig. 3 shows the secretion of IL-10 and IL-12 by immature, semi-mature, unloaded DCs and mature, survivin mRNA- as well as survivin m-RNA + peptide loaded DCs, cultured as described in the examples.

Fig. 4 shows the induction of T-cell proliferation upon incubation with immature, semi-mature, unloaded DCs and mature, survivin mRNA- as well as survivin m-RNA + peptide loaded DCs, cultured as described in the examples.

Fig. 5 shows the spontaneous and induced (by CCL19) cell migration displayed by immature, semi-mature, unloaded DCs and mature, survivin mRNA- as well as survivin m-RNA + peptide loaded DCs, cultured as described in the examples.

Fig. 6 shows the spontaneous and induced (by CCL19) phagocytosis displayed by immature, semi-mature, unloaded DCs/mRNA loaded DCs and mature, survivin mRNA-loaded DCs, cultured as described in the examples.

Fig. 7 shows the stimulation of T-cells cytotoxic and T-mem upon incubation with double-loaded DCs, whereas the T-reg number was strongly down-regulated upon incubation with double-loaded DCs.

### EXAMPLES:

Monocytes are isolated from patients' apheresate and monitored for the following markers:

| | | | |
|---|---|---|---|
| FITC | PE | PC5 | PC7 |
| CD14 | CD19 | CD3 | CD45 |
| CD41 | GPA | CD16 | CD45 |

Subsequently, PBMCs collected by leukapheresis are Ficolled, recounted and re-monitored as described before. At this point, Ficolled-cells or cells derived from elutriation are set for the adherence for 2h in Cell Gro DC, added up with GM-CSF (1000-2500 U/mL) and IL-4 (400-1000 U/mL). Afterwards, the non-adherent cells are discarded with the supernatant and the adherent cells are further cultured in Cell Gro DC, added up with GM-CSF (1000-2500 U/mL) and IL-4 (400-1000 U/mL), in a concentration of 1-2 Mio/mL for 5-6 days in order to transform the monocytes into immature DCs. Thereafter, the cells are loaded with mRNA. Shortly, DCs are washed twice in PBS, counted, and spun at 300xg for 10'. They are resuspended in CellGro DC medium and incubated in mRNA-containing solution (0.1-100 µg RNA/1x10⁶ DCs) for 2-4h in a humidified incubator at 37°C/5% CO₂. Without washing, the cultures are subsequently incubated with the proteic antigen solution (0.1-100 µg peptide/1x10⁶ DCs) for 2-12h in a humidified incubator at 37°C/5% CO₂. Thereafter, without washing, the cultures are added up with Ribomunyl (0.1-10 □g/mL) and INF-γ (400-1000 U/mL) and let mature for 8-24h. The matured DCs are stored in aliquots of at least 10 millions DCs each in Glycerol (aqueous solution) 10%.

The quality control (QC) is performed as following described:
one aliquot of cells is thawed and put in culture for 48h.

### Phenotype:

| | | | |
|---|---|---|---|
| FITC | PE | PC5 | PC7 |
| CD80 | CXCR4 | CD83 | CD45 |
| CD80 | CD19 | CD3 | CD45 |
| HLA-ABC | HLA-DR | CD40 | CD45 |

Mature DCs show the up-regulation of CXCR4, CD40, CD80, CD83, HLA-ABC and HLA-DR.

Cell viability is measured by Casy Counter with a GMP-conform software and must be at least ≥ 75%.

The contamination by T-cells and B-cells is monitored by detecting via FACS the number of CD3 and CD19 positive cells. The contamination is always kept under 25%.

### Functionality:

The immune activity test is performed through the analysis of the production of IL-12 vs IL-10 through an ISO 9002 validated ELISA assay and induction of lymphocytes proliferation via mixed leukocyte reaction as well as a tetramers activation analysis by FACS.

The phagocytosis and migration activity of the dl-DCs is monitored by FITC-dextran uptaking and Transwell-assay, respectively.

Ideally but not exclusively, the vaccine is applied intradermally, for 8 times to the patients through 2 cycles. In the first one, 6 injections are subsequently applied every week. The second cycle starts after 8 weeks of pause and comprises 2 injections, weekly administered.

### Efficacy:

The efficacy of the therapy, along with clinical evaluation, is analysed by the following parameters :
1. decrease of DTCs in the peripheral blood;
2. immune monitoring of T-cells, NKs and NKTs phenotype of activation;
3. DTH (Delayed Type Hypersensitivity reaction) test.

Patient's response is further measured by time-to-progression, reduction in the size of primary tumor and regression of metastases.

### Materials and Methods

### 1. Cell isolation

Leukoaphereses and elutriations are performed with GAMBRO equipment and following the manufacture instruction.

### 2. Ficoll purification

10 mL of blood/apheresate are mixed with 30 mL of PBS + 10% citrate buffer. Thereafter 30 mL of the mix are added to 20 mL of Ficoll and centrifuge 20' at 2000 rpm at RT. Afterwards the interfaces are collected and centrifuged 10' at 1200 rpm at RT. Finally, the pellets are resuspended in PBS, washed twice 10' at 1200 rpm at RT and resuspend in CellGro DC.

### 3. Cell culture

DCs are cultures on plastic flasks in Cell Gro DC added up with GM-CSF (400-1000 U/mL) and IL-4 (100-400 U/mL) 6 days and matured upon addition of Ribomunyl (0.5-1 µg/mL) and INF-γ (200-1000 U/mL) for 24h.

### 4. FACS

50 µL of whole blood or 50 µL of cell-suspension (thawed DCs, in a final concentration of 1 x 10⁶ cells/mL in PBS, 1%BSA, 0.1% NaN₃) are incubated 15' at room temperature with the appropriate amount of FACS antibodies, according to the manufacturer's recommendations (erythrocytes of whole blood are chemically lysed), washed at 800 rpm (RT) with 1.5 mL or 0.8 mL of PBS respectively, resuspended in 0.5 mL of PBS, supplemented with fixative and further processed by FACS®.

### 5. ELISA

Frozen DCs are thawed and re-cultured in RPMI supplemented with 2% HS at a final concentration of 1x10⁶ cells/mL. After 24h, the supernatant is harvested and analysed for human IL-10 ad IL-12p70, using CellScience ELISA KIT ISO 9002 and following the product instructions.

### 6. Mixed leukocyte reaction

Frozen DCs are thawed, and co-cultured at a final concentration of 1x10⁴ cells/well in a 96-well U-bottom plate with the non-adherent fraction of Ficoll-separated PBMNCs from a healthy donor at a final concentration of 1 x 10⁵ per cells/well in RPMI with 2% human serum (off the clott, AB), supplemented with 10% alamarBlue® at a final volume of 150 µL. Proliferation causes reduction of the dye changing from oxidized (non-fluorescent, blue) form to reduced (fluorescent, red) form. For 2-7 days, every 4h, the absorbance is measured in a plate-reader at 595 nm with reference wavelength at 620 nm.

### 7. DTCs isola tion

The DTCs isolation is performed according to the protocol provided with OncoQuick kit (Gentech).

### 8. Survivin mRNA transcription and purification

Survivin mRNA transcription and purification are performed, according to the manufacturer's protocols, through the use of mMESSAGE mMACHINE® T7/T3 Kits (Ambion) and of Oligotex mRNA Mini Kit (Qiagen), respectively.

### 9. Protein/peptide synthesis

All the used tumor specific proteins/peptides are synthesized under GMP conform rules by the American Peptide Company, Inc. or equally certified companies, which produce GMP-grade peptides for clinical use or which produce research-grade peptides, with an official certificate of analysis.

### 10. Western blot

The cells are lysed in Frackelton buffer for 20' in ice and centrifuged at 1000g 10' at 4°C. After collecting the supernatant, the protein concentration is measured with the Bradford assay and blue sample buffer is added to each sample in a ratio of 4:1.

After preparing a polyacrilamide gel, the proteins are run and blotted on a nitrocellulose membrane. Thereafter, the membrane is washed in TBS-T and incubated at first 1h in about 10 mL of blocking solution, made up of TBS-T 1X added up with 0.5 mL of Tween-20 and milk 5% and then in 10 mL of the first antibody diluted in TBS-T w/o milk, added up of 3% BSA and 0.02% NaNₛ, o/n. Thereafter the membrane is washed 3X in TBS-T and incubated with the secondary antibody for 40' at RT. Finally the protein expression is detected with the ECL kit.

### Results

All the results showed in this section were performed on DCs, cultured as described previously and frozen in Glycerol 10%. Cell viability, after thawing, was >75%. The results obtained with 10% Glycerol solution are comparable or slightly superior to those showed upon freezing with DMSO 10%. Freezing cells in glycerol has the major advantage on freezing them with DMSO that it does not require a centrifuging step to eliminate toxic substances (DMSO) and therefore it allows an easier way to inject the DCs into the patients, without special tools and equipment (centrifuge, lamina flow, steril environment) requirement.

To grant that DCs incubated with survivin mRNA and protein antigen (PSA) were indeed loaded, a Western Blot to determine whether the phagocyted mRNA was as well translated and whether the protein antigen was expressed by the DCs was performed.

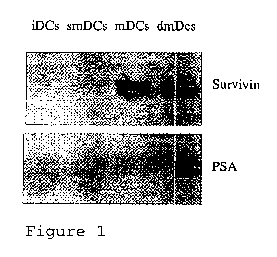

1 Mio cells were washed twice in PBS, lysed in Frackelton buffer and processed by Western Blot. The results reported in Fig. 1 show that immature DCs (iDCs) and unloaded, semi-mature DCs (smDCs) do not express neither survivin nor PSA. Mature DCs, loaded with survivin mRNA (mDCs), shows the expression of this antigen but not of PSA and mature DCs, loaded with survivin and PSA (dmDCs), show the expression of both antigens. These results confirm that the loading procedure is functional for both mRNA and protein loading and it is very efficient, without the need of carriers or active transfection means.

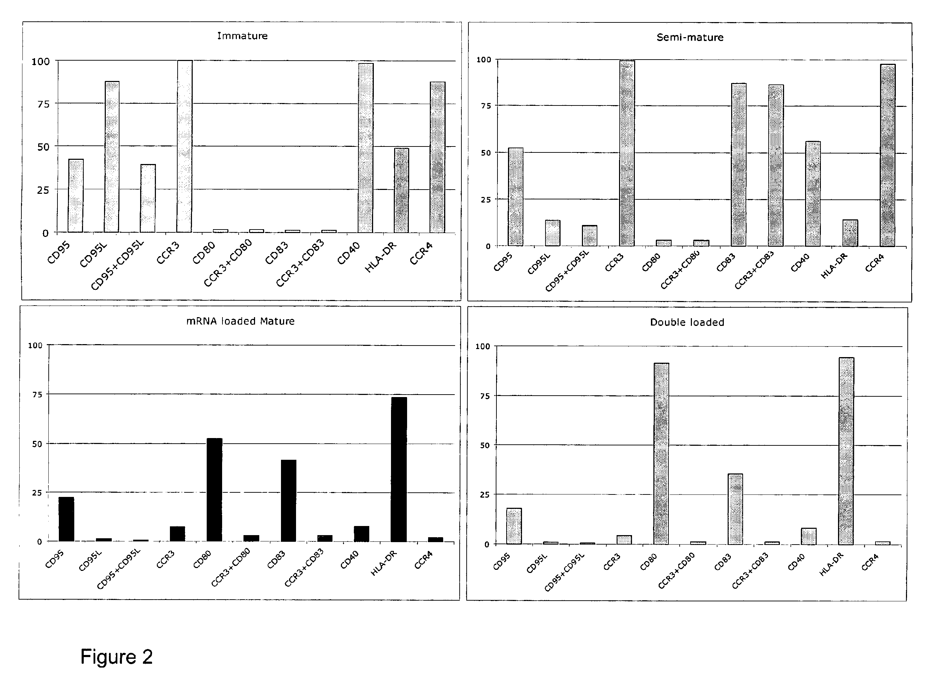

In 10 patients it has been shown that DCs, cultured as described in the present protocol expressed different CDs groups on their surface, according to the different phases of maturation. Not-stimulated DCs do not express maturing markers such as CD80 and CD83, but expressed immature markers, such as CCR3. Semi-mature, unloaded DCs show a combination of immature markers, such as CCR3 as well as low CD80, and of mature markers, such as CD83. Fully mature DCs, loaded with survivin mRNA showed only mature markers, such as CD80 and CD83, but no immature markers, such as CCR3 (Fig. 2).

To determine whether the present protocol allows not only the phenotypic but as well the functional maturation of the DCs, the secretion of IL-10 and IL-12 in the supernatant was analysed. IL-12 is known to enhance the ability of the immune system to kill tumor cells and may interfere with blood flow to the tumor. IL-12 enhances tumor clearance by stimulating the activation of the cytotoxic T-cell. On the other side, IL-10 has been shown to have a significant inhibitory effect on several aspects of DC function; such as the expression of costimulatory molecules and the ability to synthesize IL-12. Importantly, IL-10-treated DCs become tolerogenic. Normally, iDCs do not produce neither IL-10 (or very slightly) nor IL-12, whereas mDCs secrete high levels of IL-12 and very low levels of IL-10.

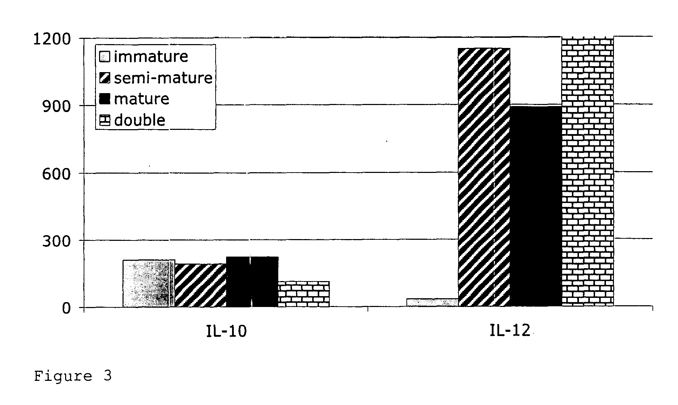

As expected, iDCs did not produce IL-12 and only a minimal quantity of IL-10, whereas semimature, unloaded DCs, survivin mRNa loaded, mature DCs and survivin mRNA + peptide loaded, mature DCs cultured following our protocol produce high levels of IL-12 and very low levels of IL-10 (Fig. 3).

To further demonstrate that the DCs according to the present invention are functional active, their effects on heterologous T-cell proliferation through a mix-leukocyte reaction is assessed.

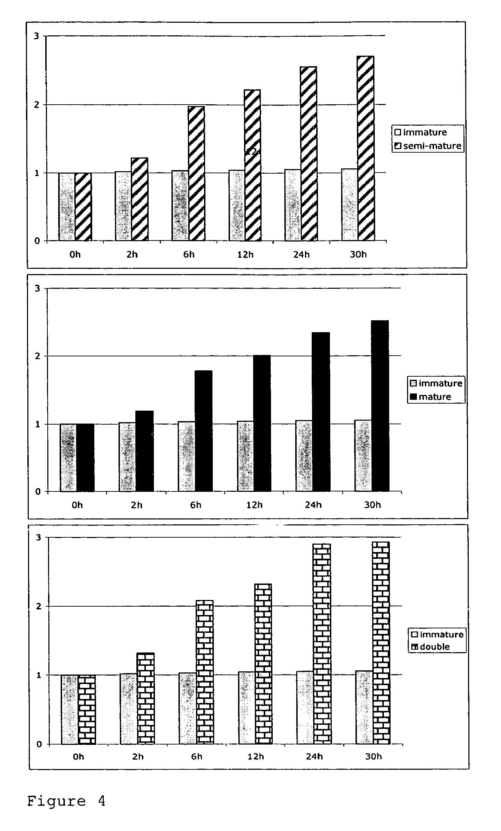

Incubation of semi-mature, unloaded and mature, survivin mRNA/survivin mRNA + peptideloaded DCs increased proliferation of T-cells, showing that DCs cultured as described in the present example display a high positive effect on at least one of the major players of the immunosystem (T-cells), showing a potential benefit on tumor clearence. As expected, incubation of iDCs with T-cells did not increase their proliferation rate (Fig. 4).

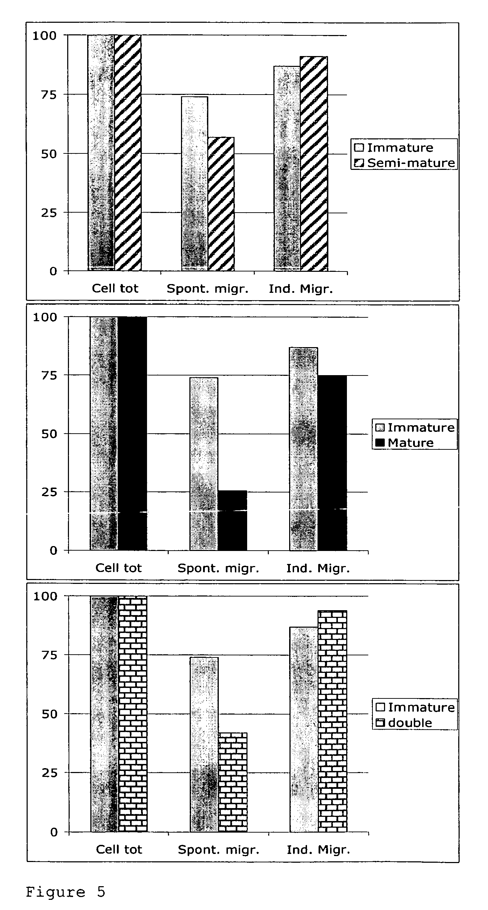

iDCs are characterized by a strong migration potentiality, which is not inducible by CCL19 addition due to lack of CXCR4/CCR7 expression on iDCs. Semi-mature DCs, on the contrary, respond to CCL19 induction but still retain spontaneous migration ability, even if to less extend, compared to iDCs. Mature, loaded DCs (almost) completely lose the capability to migrate spontaneously but they do migrate upon CCL19 induction (Fig. 5). This means that semimature unloaded DCs as well as mature, mRNA/survivin mRNA + peptide-loaded DCs are able to move from the injection site towards the stimulus source (i.e. tumor site) and therefore they need not be injected directly intranodal, but they can as well be injected in the surrounding of the tumor. This fact makes easier the use of the DC vaccine, since it does not require specific tools, such as ultrasound device, to exactly determine the node position.

Finally it was investigated whether DCs cultured as described in the protocol were still able to actively phagocyte spontaneously or in response to CCL19.

iDCs are characterized by a strong phagocytotic capability, which is not inducible by CCL19, due to lack of CXCR4/CCR7 expression on iDCs. Semi-mature DCs and survivin mRNA-loaded DCs, on the contrary, respond to CCL19 induction but still retain spontaneous phagocytosis ability, even if to less extend compared to iDCs. Mature, survivin mRNA and peptide loaded DCs completely lose the capability to phagocyte both spontaneously and upon CCL19 induction.

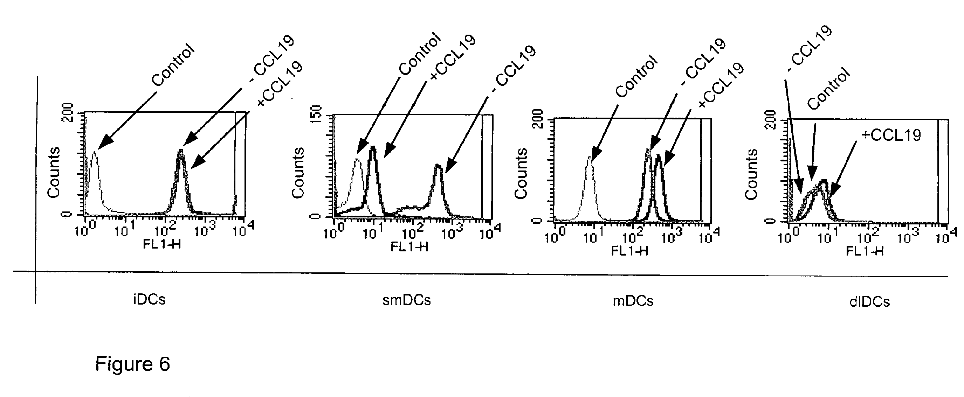

This means that semi-mature, unloaded DCs as well as mRNA loaded DCs, prepared following the protocol of the present invention are ready to phagocyte tumor antigen, showing a very active phenotype that may strongly increase the direct immune response to the tumor, while double loaded (mRNA Cell + peptide) and fully mature DCs are ready to induce T-cell proliferation and activation but lost their ability to further phagocyte tumor tissue, increasing the specificity of the immune response towards the already uptaken antigen (Fig. 6).

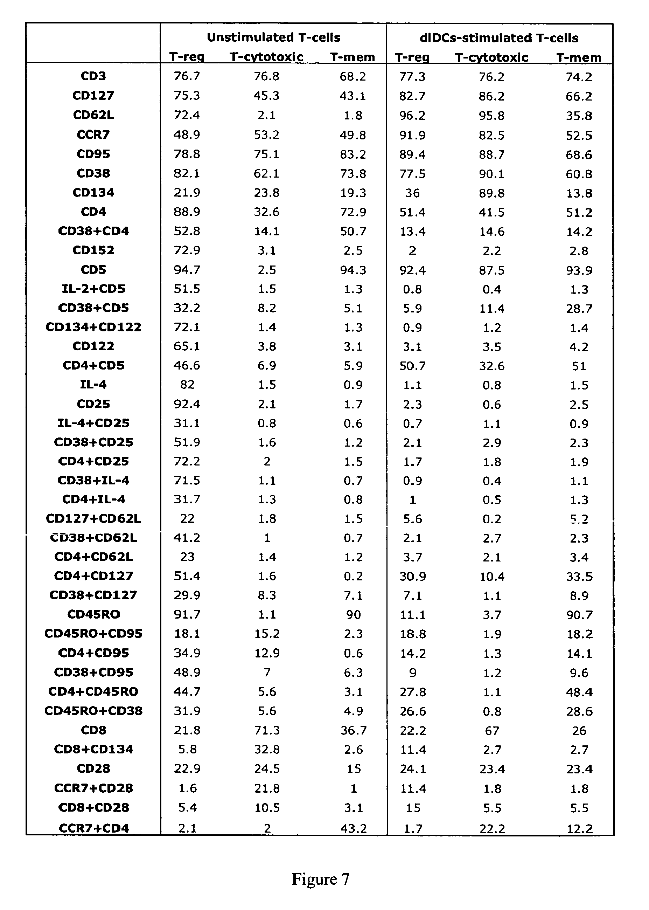

Finally, the double loaded DCs, with mRNA and protein, were shown able to induce a high cytotoxic and memory response without triggering the activation of T-reg response, upon in vitro incubation with allogen lymphocytes (Fig. 7).

## Claims

1. Dendritic cell loaded with at least one nucleic acid molecule encoding a tumor associated antigen or fragment thereof and at least one proteinaceous molecule being a tumor associated antigen or fragment thereof.

2. Cell according to claim 1, **characterised in that** the tumor associated antigen or fragment thereof encoded by the at least one nucleic acid molecule is selected from the group consisting of survivin and telomerase reverse transcriptase (TERT).

3. Cell according to claim 1 or 2, **characterised in that** the nucleic acid molecule is a ribonucleic acid, preferably mRNA, and/or a desoxyribonucleic acid.

4. Cell according to any one of claims 1 to 3, **characterised in that** the tumor associated antigen is selected from the group consisting of cancer type-specific tumor associated antigens, preferably carcinoembryonic antigen (CEA), mucin (MUC), prostate-specific membrane antigen (PSMA), BRCA and ras, and/or a universal tumor associated antigen selected from the group consisting of survivin and TERT.

5. Cell according to any one of claims 1 to 4, **characterised in that** the dendritic cells are of human or animal origin.

6. Cell according to any one of claims 1 to 5, **characterised in that** the dendritic cells are mature.

7. Method for manufacturing dendritic cells loaded with at least one nucleic acid molecule encoding a tumor associated antigen or fragment thereof and at least one proteic molecule being a tumor associated antigen or fragment thereof, preferably according to any one of claims 1 to 6, comprising the steps of:
- providing dendritic cells and
- loading said cells with at least one nucleic acid molecule encoding a tumor associated antigen or fragment thereof and at least one proteic molecule being a tumor associated antigen or fragment thereof.

8. Method according to claim 7, **characterised in that** dendritic cells are provided by transforming monocytes into dendritic cells

9. Method according to claim 8, **characterised in that** the loaded immature dendritic cells are matured.

10. Dendritic cells obtainable by a method according to any one of claims 7 to 9.

11. Pharmaceutical preparation comprising dendritic cells according to any one of claims 1 to 6 or dendritic cells according to claim 10.

12. Preparation according to claim 11, **characterised in that** the preparation comprises further at least one pharmaceutical excipient, at least one immunostimmulatory agent, at least one adjuvant.

13. Use of dendritic cells according to any one of claims 1 to 6 or dendritic cells according to claim 10 for the manufacture of a vaccine for treating or preventing cancer in individuals.
